Europäisches Patentamt

European Patent Office

Office européen des brevets

(19)

(11) **EP 1 157 731 A1**

(12) **DEMANDE DE BREVET EUROPEEN**

(43) Date de publication:
**28.11.2001 Bulletin 2001/48**

(51) Int Cl.⁷: **B01D 53/04**

(21) Numéro de dépôt: **01401272.8**

(22) Date de dépôt: **16.05.2001**

(84) Etats contractants désignés:
**AT BE CH CY DE DK ES FI FR GB GR IE IT LI LU
MC NL PT SE TR**
Etats d'extension désignés:
**AL LT LV MK RO SI**

(30) Priorité: **25.05.2000 FR 0006697**

(72) Inventeurs:
• **Dubois, Anne**
  **78150 Le Chesnay (FR)**
• **Bodelin, Pierre**
  **92170 Vanves (FR)**
• **Vigor, Xavier**
  **Chicago, IL 60611 (US)**

(71) Demandeurs:
• **L'AIR LIQUIDE, SOCIETE ANONYME POUR
  L'ETUDE ET L'EXPLOITATION DES PROCEDES
  GEORGES CLAUDE
  75007 Paris (FR)**
• **Air Liquide Sante (International)
  75341 Paris Cedex 07 (FR)**

(74) Mandataire: **Pittis, Olivier et al
  L'Air Liquide, S.A.,
  Service Brevets & Marques,
  75, Quai d'Orsay
  75321 Paris Cedex 07 (FR)**

(54) **Concentrateur d'oxygène portable**

(57)    L'invention concerne un concentrateur oxygène portable par un patient permettant de produire un flux gazeux contenant de 50 % à 95 % d'oxygène à partir d'air comprenant des moyens de compression d'air, des moyens de séparation de gaz par adsorption avec variations de pression, et des moyens accumulateurs d'énergie électrique ayant une autonomie d'au moins 30 minutes, ledit concentrateur ayant une masse totale inférieure à 10 kg. De préférence, les moyens de séparation de gaz sont un système PSA utilisant une zéolite X échangée au lithium en tant qu'adsorbant.

FIG.1

EP 1 157 731 A1

**Description**

L'invention concerne un concentrateur d'oxygène portable utilisable en oxygénothérapie.

Les concentrateurs oxygène utilisant la technique PSA (Pressure Swing Adsorption = Adsorption avec variations de pression) sont actuellement très répandus pour l'oxygénothérapie à domicile. Néanmoins, leur conception a un inconvénient majeur, à savoir son manque de mobilité.

En effet, les concentrateurs existants nécessitent une source d'électricité et sont de plus trop lourds pour être transportés ou portés par le patient.

Or, un certain nombre de patients sous oxygénothérapie souhaitent pourtant mener une vie la plus « normale » possible, ce qui implique en particulier de pouvoir déambuler ou se déplacer plus facilement.

Pour fournir à ces patients une solution leur permettant des déplacements de courte durée, les documents WO-A-98/58219 et US-A- 5 893 275 proposent de coupler le concentrateur de type PSA avec un liquéfacteur, de manière à remplir un Dewar (récipient) que le patient peut transporter. Cette solution est en fait plus complexe qu'il n'y paraît. Ainsi le Dewar de stockage de l'oxygène doit être réchauffé périodiquement pour éliminer toute trace d'hydrocarbures et d'eau. D'autre part, le réglage de la température de liquéfaction doit être précis de manière à éviter qu'au début de la vaporisation pour utilisation par le patient, le gaz initial ait une concentration en azote élevée.

Une autre solution proposée dans le document US-A-5 858 062 est de comprimer une partie de l'oxygène sortant du concentrateur de type PSA pour remplir une bouteille portable. Mais, il s'agit d'une solution coûteuse car basée sur l'utilisation d'un compresseur d'oxygène, et peu satisfaisante du point de vue de la sécurité puisque le patient doit manipuler de l'oxygène sous pression.

La présente invention a alors pour objectif de proposer aux patients souhaitant disposer d'une réelle mobilité une solution alternative plus simple et plus satisfaisante en ce qui concerne la sécurité, c'est-à-dire d'améliorer les solutions connues de l'art antérieur.

La présente invention concerne alors un concentrateur d'oxygène portable par un patient permettant de produire un flux gazeux contenant de 50% à 95% d'oxygène à partir d'air, comprenant :

- des moyens de compression d'air pour comprimer l'air à une pression supérieure à la pression atmosphérique (1 bar),
- des moyens de séparation de gaz par adsorption avec variations de pression pour séparer l'air comprimé par les moyens de compression d'air et produire un gaz enrichi en oxygène, et
- des moyens accumulateurs d'énergie électrique ayant une autonomie d'au moins 30 minutes permettant de stocker et fournir ou restituer de l'électricité,

- ledit concentrateur ayant une masse totale inférieure à 10 kg, et
- la masse des moyens de compression (Mcomp), la masse des moyens de séparation de gaz (Mpsa) et la masse des moyens accumulateurs d'énergie (Mpile) étant telles que :

$$0,5 < \frac{Mcomp}{Qp} < 3$$

$$0.15 < \frac{Mpile}{Qp} < 2$$

$$0,05 < \frac{Mtamis}{Qp} < 1$$

où Qp est le débit de production d'oxygène du concentrateur (en l/min) et les masses Mcomp, Mpile et Mtamis sont exprimées en kg.

Selon un autre aspect, l'invention concerne aussi un concentrateur d'oxygène portable par un patient permettant de produire un flux gazeux contenant de 50% à 95% d'oxygène à partir d'air, comprenant :

- des moyens de compression d'air pour comprimer l'air à une pression comprise entre 1 et 5 bars,
- des moyens de séparation de gaz par adsorption avec variations de pression comprennent plusieurs adsorbeurs contenant chacun un ou plusieurs adsorbants et fonctionnant selon des cycles PSA, la durée de chaque cycle de production étant inférieure à 30 secondes et au moins un adsorbant étant une zéolite échangée par au moins un cation métallique choisi parmi le lithium, le calcium, le zinc, le cuivre et leurs combinaisons,
- des moyens accumulateurs d'énergie électrique ayant une autonomie d'au moins 30 minutes,
- ledit concentrateur ayant une masse totale inférieure à 10 kg, et
- la masse des moyens de compression (Mcomp), la masse des moyens de séparation de gaz (Mpsa) et la masse des moyens accumulateurs d'énergie (Mpile) étant telles que :

$$0,5 < \frac{Mcomp}{Qp} < 3$$

$$0,15 < \frac{Mpile}{Qp} < 2$$

$$0,05 < \frac{Mtamis}{Qp} < 1$$

où Qp est le débit de production d'oxygène du concentrateur (en l/min) et les masses Mcomp, Mpile et Mtamis sont exprimées en kg,

- lesdits moyens de compression d'air, lesdits moyens de séparation de gaz par adsorption et lesdits moyens accumulateurs d'énergie électrique étant insérés à l'intérieur d'au moins un boîtier,
- ledit boîtier comprenant, en outre, des moyens de contrôle ou de réglage du fonctionnement du concentrateur et au moins un système de fixation ou de port du concentrateur.

Selon le cas, le concentrateur de l'invention peut comprendre l'une ou plusieurs des caractéristiques suivantes :

- Qp est compris entre 0,5 et 4 l/min, de préférence entre 0,5 et 2 l/min.
- le rapport (Mcomp / Qp) est compris entre 0,5 et 2 kg/(l/min).
- le rapport (Mpile / Qp) est compris entre 0,15 et 1,2 kg/(l/min).
- le rapport (Mtamis / Qp) est compris entre 0,05 et 0,8 kg/(l/min).
- Mcomp + Mpile + Mtamis ≤ 8 kg, de préférence Mcomp + Mpile + Mtamis ≤ 5 kg.
- les moyens de séparation de gaz comprennent plusieurs adsorbeurs contenant chacun un ou plusieurs adsorbants et fonctionnant selon des cycles PSA, de préférence la durée de chaque cycle de production est inférieure à 30 secondes, de préférence inférieure à 20 secondes.
- l'adsorbant a une granulométrie inférieure à 1 mm et/ou comporte des particules de zéolite X échangée par au moins un cation métallique choisi parmi le lithium, le calcium, le zinc, le cuivre et leurs combinaisons, de préférence une zéolite X ayant un rapport Si/Al d'environ 1 à 1,25 et échangée à au moins 80% par des cations lithium.
- les moyens de compression sont adaptés ou commandés pour comprimer l'air à une pression comprise entre 1 et 5 bars, de préférence entre 2,5 et 3,5 bars.
- il comporte des moyens de régulation de la température permettant d'ajuster la température de l'air d'alimentation et/ou des adsorbeurs à une valeur comprise entre 10 et 60°C.
- les moyens de contrôle ou de réglage du fonctionnement du concentrateur comportent au moins un moyen de marche/arrêt pour mettre en fonction ou arrêter le fonctionnement du concentrateur, de préférence le moyen de marche/arrêt comprend un bouton d'actionnement, ou un organe de commande actionnable par l'opérateur.
- le système de fixation ou de port du concentrateur comprend au moins une poignée de portage et/ou au moins une bandoulière ou une sangle et/ou au moins un système d'accrochage à la ceinture.
- il comprend des moyens de réglage du débit de gaz à produire par les moyens de séparation de gaz par adsorption.

La présente invention va maintenant être mieux comprise grâce à la description détaillée suivante faite en références aux figures annexées.

Contrairement à ce qu'affirme le document WO-A-98/58219, les inventeurs de la présente invention ont montré qu'il est en fait possible de réaliser un concentrateur réellement portable, à condition de combiner un certain nombre d'avancées techniques qui vont être détaillées ci-dessous , à savoir un cycle court de production, une granulométrie d'adsorbant faible, un adsorbant « performant », et une utilisation d'un système permettant de réduire le débit à produire par le concentrateur tout en satisfaisant les besoins en oxygène du patient

Il s'ensuit alors qu'un concentrateur $O_2$ pourra être considéré comme portable si les deux conditions suivantes sont vérifiées, à savoir une masse m inférieure à 10 kg, de préférence inférieure à 7 kg, et s'il peut fonctionner sur batteries, avantageusement rechargeables, ayant une autonomie en courant électrique d'au moins 30 minutes, de préférence d'au moins 1 heure et préférentiellement encore d'au moins 2 heures.

Or, la masse totale (MT) d'un concentrateur PSA dépend du débit produit et des performances du cycle :

- rendement η = $O_2$ produit/$O_2$ entrant
- productivité par cycle Pcy = $O_2$ produit/cycle/$m^3$ d'adsorbant
- temps de cycle Tcy = durée d'un cycle de production (en secondes).

Elle dépend bien entendu aussi des « performances massiques » des différents composants, par exemple du rapport entre la masse du compresseur et le débit d'air qu'il comprime.

La mise au point d'un concentrateur portable passe donc par une étape d'établissement des relations entre performances du PSA et masses des différents composants. L'efficacité du système sera mesurée par la masse nécessaire pour produire 1 l/min d'oxygène. Le système sera d'autant plus léger que ce rapport sera faible et/ou que le débit d'oxygène demandé sera faible.

Les principaux composants dont la masse doit être réduite sont le compresseur d'air, l'adsorbant et la pile ou les moyens d'accumulation ou de courant électrique mis en oeuvre.

Il existe, bien sûr d'autres constituants de l'appareil (carcasse externe, adsorbeurs du système PSA, canalisations internes, vannes...) mais leur masse est faible, voire négligeable par rapport à celle des principaux constituants.

Compresseur d'air

Le débit d'air (Qa) devant être fourni par le compresseur est

$$Q_a = \frac{Q_p}{\eta \times 0.21}$$

où

Qp est le débit d'oxygène produit (en l/min)
η est le rendement défini ci-dessus.

Or, les meilleurs compresseurs disponibles ont une « efficacité massique » comprise entre 1 kg pour 5 l/min et 1 kg pour 10 l/min. Ces valeurs permettent donc de tracer deux courbes permettant d'encadrer le rapport : Masse compresseur/Débit d'oxygène produit fonction du rendement.

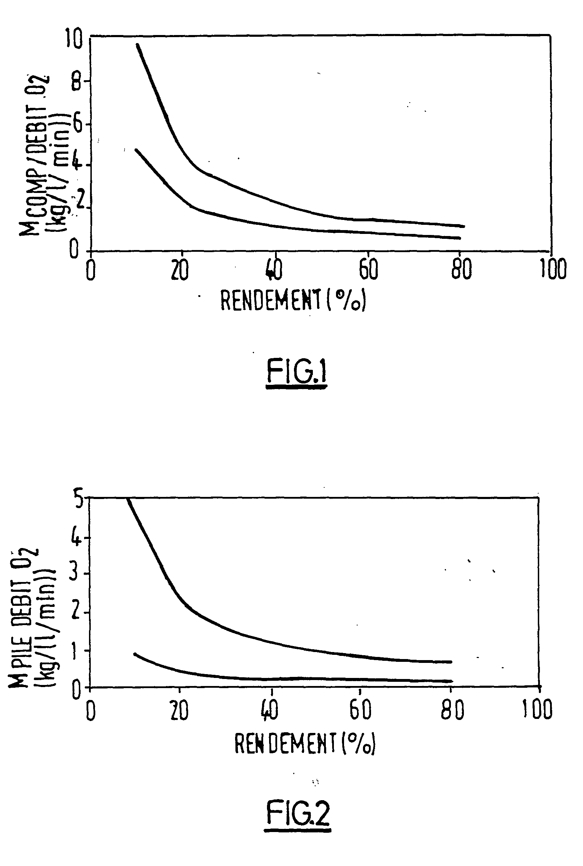

Ces courbes sont schématisées sur la figure 1.

Partant des courbes de la figure 1 et sachant que les rendements obtenus pour un cycle PSA sont typiquement compris entre 30 et 60%, on peut établir l'inégalité suivante : 0.5 < Mcomp/Qp < 3 kg (en kg/(l/min))

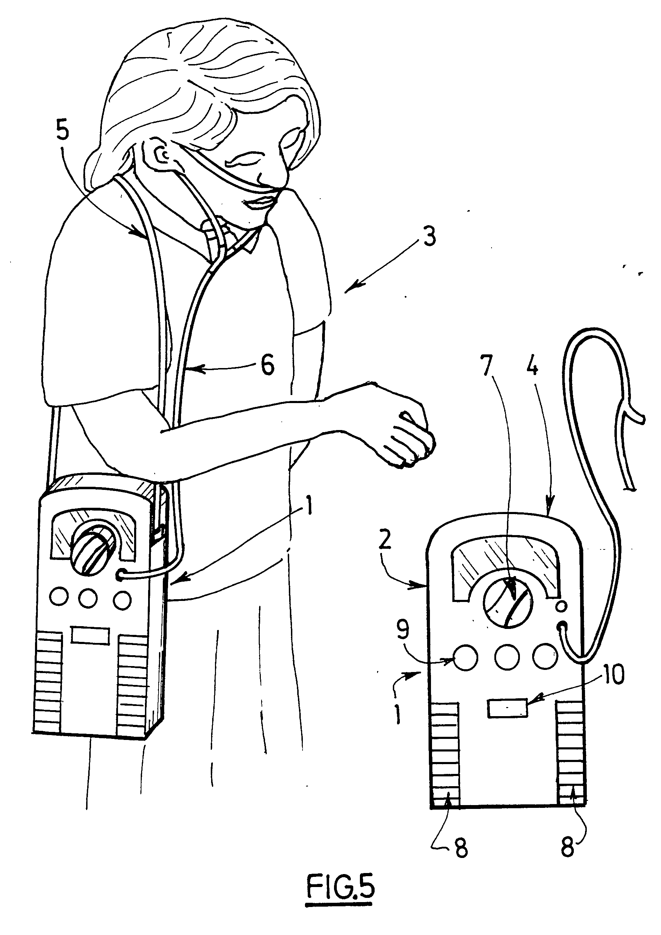

Pile/batterie (réf. 8 sur fig. 5)

Par analogie, l'énergie spécifique Es (en KWh/l d'oxygène produit) d'un système PSA peut s'exprimer par la relation suivante :

$$E_s = \frac{k}{\eta *0.21} \times \log(\frac{Ph}{Patm})$$

où

Ph est la pression haute du cycle (en bar)
k est comprise entre 0.11 et 0.15 suivant le compresseur
Patm est la pression atmosphère (1 bar)

Pour une autonomie de 2 heures, l'énergie nécessaire (en Wh) s'exprime alors par la relation suivante :

$$E = \frac{k}{\eta \times 0.21} \times Q_p \times 120 \times \log(Ph/Patm)$$

La pression haute d'un système PSA étant classiquement comprise entre 2.5 et 3.5 bars et l'efficacité massique des meilleures piles entre 1 kg pour 100 Wh et 1 kg pour 300 Wh, on peut là encore tracer deux courbes (représentées en figure 2) permettant d'encadrer le rapport masse pile/débit O2 fonction du rendement. On obtient l'inégalité suivante : 0.15< Mpile/Qp < 2 (exprimé en kg/(l/min))

Masse d'adsorbant du PSA

De même, la masse d'adsorbant (Mads) est donnée par la relation suivante :

$$Mads = \frac{T_{cy} \times Q_p \times \rho_{ads}}{P_{cy}}$$

où $\rho_{ads}$ est la masse volumique de l'adsorbant, typiquement comprise entre 0.5 et 0.7 kg/l. Tcy et Pcy sont comme donné ci-dessus.

Les productivités par cycle typiquement obtenues dans un cycle PSA sont comprises entre 0.2 et 0.5 Nl/h/l. La masse d'adsorbant est directement proportionnelle au temps de cycle. La réduction du temps de cycle passe par une réduction de la granulométrie de l'adsorbant pour améliorer la cinétique d'adsorption. Les temps de cycle des concentrateurs médicaux sont en général inférieurs à 25 s grâce à l'utilisation d'un adsorbant de granulométrie moyenne inférieure à 1 mm. Ils peuvent descendre à quelques secondes, comme indiqué par le document US-A- 5,827,358.

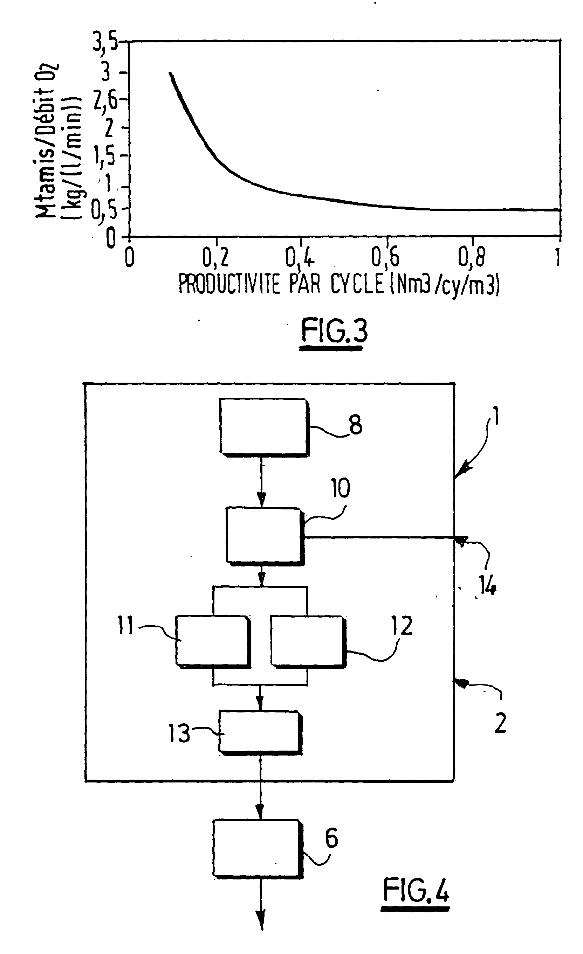

On obtient, là encore, deux courbes permettant d'encadrer le rapport Mads/Débit O$_2$ produit fonction de la productivité du PSA (productivité par cycle), comme représenté en figure 3.

De là, on obtient l'inégalité suivante :
0.05 < Mtamis/Qp < 1 (exprimée en kg/(l/min))

Le reste du matériel permettant de réaliser le concentrateur a une masse relativement peu dépendante du débit de production et peut être estimée à 1 ou 2 kg au maximum.

Les courbes des figures 1 à 3 mettent en évidence trois voies de réduction de la masse d'un concentrateur :

- réduction du débit moyen exigé Qp
- augmentation des performances massiques des composants: compresseur, pile...
- augmentation des performances du procédé PSA.

L'augmentation des performances massiques des composants est du ressort des fabricants. Dans la présente invention, on se contente de choisir des composants se situant dans les limites de masse précisées ci-dessus.

La réduction du débit moyen exigé Qp passe, pour satisfaire les besoins en oxygène du patient, par l'adjonction préférentielle d'un système à valve économiseuse permettant de délivrer l'oxygène au patient de manière synchronisée avec la respiration et donc de diviser la production nécessaire d'oxygène du concentrateur par un facteur compris entre 1.5 et 6, de préférence compris entre 2 et 4.

La prescription habituelle d'oxygène gazeux pour un patient en oxygénothérapie est comprise entre 3 et 6 l/min. L'utilisation d'une telle vanne économiseuse permet donc de réduire le débit d'oxygène moyen devant être produit par le concentrateur à une valeur comprise entre 0.5 et 4 l/min de préférence entre 0.5 et 2l/min.

L'augmentation des performances du procédé PSA est obtenue par :

- utilisation d'un adsorbant performant, de préférence une zéolite X échangée au lithium permettant d'obtenir un rendement supérieur à 45 % et une productivité par cycle supérieure à 0.3 Nm3/h,
- temps de cycle inférieur à 20 s, de préférence inférieur à 15 s.

Dans ce cas, les inégalités précédentes deviennent alors :

$$0.5 < Mcomp/Qp < 2 \qquad kg/(l/min)$$
$$0.1 < Mpile/Qp < 1.2 \qquad kg/(l/min)$$
$$0.05 < Mtamis/Qp < 0.8 \qquad kg/(l/min)$$

Dans ces conditions, on constate que la somme des masses des différents composants sera inférieure à 8 kg pour des valeurs de débit moyen allant jusqu'à 2 l/min.

De façon générale, comme montré sur la figure 5, un concentrateur portable 1 selon l'invention se présente dans un boîtier 2 de taille et de poids permettant au patient 3 de le garder sur lui, tout en déambulant.

Les systèmes de fixation ou de port du concentrateur 1 par le patient 3 possibles sont une poignée 4 et/ou une bandoulière 5, prévues sur le concentrateur 1 directement ou sur une sacoche dimensionnée à cet effet, qui permet de le protéger pour toute utilisation en extérieur.

Le boîtier 2 est stable et peut se poser sur toute surface plane. Il présente avantageusement :

- une sortie d'air enrichi en $O_2$, qui peut se connecter au moyen d'administration 6 du gaz vers le patient 3 ;
- un bouton 9 marche/arrêt;
- un dispositif de réglage 7 du débit de production d'air enrichi en $O_2$;
- un écran 10 et/ou autre système de visualisation (voyants lumineux par exemple) permettant d'informer le patient ou toute autre personne, des réglages consignés ainsi que des risques potentiels et/ou des dysfonctionnements (autonomie résiduelle du moyen d'alimentation électrique, moyen de compression défaillant par exemple) ;
- des étiquettes d'information garantissant la qualité du matériel et éventuellement les recommandations essentielles à son maintien en bon état de marche.

La figure 4 schématise le principe de fonctionnement d'un concentrateur 1 selon l'invention comportant un boîtier 2 externe dans lequel sont incorporés une ou des entrées 14 d'air ambiant (systèmes d'ouies par exemple) permettant d'alimenter les moyens de compression 10 de gaz, et de générer éventuellement une circulation d'air dans le boîtier 2, des moyens de compression de gaz 10, des moyens de séparation de gaz par adsorption avec variations de pression (PSA) comprenant plusieurs adsorbeurs 11, 12 renfermant des particules d'adsorbant, des moyens accumulateurs 8 d'énergie électrique, une capacité tampon 13 pour stocker l'air enrichi en oxygène produit, et des moyens d'amenée 6 de l'air enrichi en oxygène produit vers les voies respiratoires du patient 3.

En outre, aux éléments majeurs constituant le concentrateur schématisé en figure 4 viennent s'ajouter :

- un ou plusieurs moyens de filtration (poussières, antibactériens...) de l'air ambiant et/ou de l'air enrichi en $O_2$;
- une carte électronique de gestion des différents composants et de leurs alarmes ;
- un système d'insonorisation de l'ensemble (par exemple de la mousse) et plus particulièrement du compresseur, via des silent blocks par exemple.

Préférentiellement, l'adsorbant utilisé dans le système PSA est un adsorbant, de préférence de type zéolites X ou LSX échangées à plus de 80 % au lithium dû type de celles décrites dans le document EP-A-785020.

## Revendications

1. Concentrateur d'oxygène portable par un patient permettant de produire un flux gazeux contenant de 50% à 95% d'oxygène à partir d'air, comprenant :

   - des moyens de compression d'air,
   - des moyens de séparation de gaz par adsorption avec variations de pression, et
   - des moyens accumulateurs d'énergie électrique ayant une autonomie d'au moins 30 minutes,
   - ledit concentrateur ayant une masse totale inférieure à 10 kg, et
   - la masse des moyens de compression (Mcomp), la masse des moyens de séparation de gaz (Mpsa) et la masse des moyens accumulateurs d'énergie (Mpile) étant telles que :

$$0,5 < \frac{Mcomp}{Qp} < 3$$

$$0,15 < \frac{Mpile}{Qp} < 2$$

$$0,05 < \frac{Mtamis}{Qp} < 1$$

   où Qp est le débit de production d'oxygène du concentratèur (en l/min) et les masses Mcomp, Mpile et Mtamis sont exprimées en kg.

**2.** Concentrateur selon la revendication 1, **caractérisé en ce que** Qp est compris entre 0,5 et 4 l/min, de préférence entre 0,5 et 2 l/min.

**3.** Concentrateur selon la revendication 1 ou 2, **caractérisé en ce que** le rapport (Mcomp / Qp) est compris entre 0,5 et 2 kg/(l/min).

**4.** Concentrateur selon les revendications 1 à 3, **caractérisé en ce que** le rapport (Mpile / Qp) est compris entre 0,15 et 1,2 kg/(l/min).

**5.** Concentrateur selon les revendications 1 à 4, **caractérisé en ce que** le rapport (Mtamis / Qp) est compris entre 0,05 et 0,8 kg/(l/min).

**6.** Concentrateur selon les revendications 1 à 5, **caractérisé en ce que** Mcomp + Mpile + Mtamis $\leq$ 8 kg, de préférence Mcomp+Mpile+Mtamis $\leq$ 5 kg.

**7.** Concentrateur selon les revendications 1 à 6, **caractérisé en ce que** les moyens de séparation de gaz comprennent plusieurs adsorbeurs contenant chacun un ou plusieurs adsorbants et fonctionnant selon des cycles PSA, de préférence la durée de chaque cycle de production est inférieure à 30 secondes.

**8.** Concentrateur selon les revendications 1 à 7, **caractérisé en ce que** l'adsorbant a une granulométrie inférieure à 1 mm et/ou comporte des particules de zéolite X échangée par au moins un cation métallique choisi parmi le lithium, le calcium, le zinc, le cuivre et leurs combinaisons, de préférence une zéolite X ayant un rapport Si/Al d'environ 1 à 1,25 et échangée à au moins 80% par des cations lithium.

**9.** Concentrateur selon les revendications 1 à 8, **caractérisé en ce que** les moyens de compression sont adaptés ou commandés pour comprimer l'air à une pression comprise entre 1 et 5 bars, de préférence entre 2,5 et 3,5 bars.

**10.** Concentrateur selon les revendications 1 à 9, **caractérisé en ce qu'**il comporte des moyens de régulation de la température permettant d'ajuster la température de l'air d'alimentation et/ou des adsorbeurs à une valeur comprise entre 10 et 60°C.

**11.** Concentrateur d'oxygène portable par un patient permettant de produire un flux gazeux contenant de 50% à 95% d'oxygène à partir d'air, comprenant :

- des moyens de compression d'air pour comprimer l'air à une pression comprise entre 1 et 5 bars,

- des moyens de séparation de gaz par adsorption avec variations de pression comprennent plusieurs adsorbeurs contenant chacun un ou plusieurs adsorbants et fonctionnant selon des cycles PSA, la durée de chaque cycle de production étant inférieure à 30 secondes et au moins un adsorbant étant une zéolite échangée par au moins un cation métallique choisi parmi le lithium, le calcium, le zinc, le cuivre et leurs combinaisons,

- des moyens accumulateurs d'énergie électrique ayant une autonomie d'au moins 30 minutes,

- ledit concentrateur ayant une masse totale inférieure à 10 kg, et

- la masse des moyens de compression (Mcomp), la masse des moyens de séparation de gaz (Mpsa) et la masse des moyens accumulateurs d'énergie (Mpile) étant telles que :

$$0,5 < \frac{Mcomp}{Qp} < 3$$

$$0,15 < \frac{Mpile}{Qp} < 2$$

$$0,05 < \frac{Mtamis}{Qp} < 1$$

où Qp est le débit de production d'oxygène du concentrateur (en l/min) et les masses Mcomp, Mpile et Mtamis sont exprimées en kg,

- lesdits moyens de compression d'air, lesdits moyens de séparation de gaz par adsorption et lesdits moyens accumulateurs d'énergie électrique étant insérés à l'intérieur d'au moins un boîtier,

- ledit boîtier comprenant, en outre, des moyens de contrôle ou de réglage du fonctionnement du concentrateur et au moins un système de fixation ou de port du concentrateur.

**12.** Concentrateur selon la revendication 11, **caractérisé en ce que** les moyens de contrôle ou de réglage du fonctionnement du concentrateur comportent au moins un moyen de marche/arrêt pour mettre en fonction ou arrêter le fonctionnement du concentrateur, de préférence le moyen de marche/arrêt comprend un bouton d'actionnement ou un organe de commande actionnable par l'opérateur.

**13.** Concentrateur selon l'une des revendications 11 ou 12, **caractérisé en ce que** le système de fixation ou de port du concentrateur comprend au moins une poignée de portage et/ou au moins une bandoulière ou une sangle et/ou au moins un système d'accrochage à la ceinture.

**14.** Concentrateur selon l'une des revendications 1 ou 11, **caractérisé en ce qu'**il comprend des moyens de réglage du débit de gaz à produire par les moyens de séparation de gaz par adsorption.

FIG.1

FIG.2

FIG.3

FIG.4

FIG.5

**Office européen des brevets**

## RAPPORT DE RECHERCHE EUROPEENNE

Numéro de la demande

EP 01 40 1272

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 4 971 609 A (R.A.PAWLOS) 20 novembre 1990 (1990-11-20) * le document en entier * --- | 1,7 | B01D53/04 |
| A | US 4 491 459 A (C.J.PINKERTON) 1 janvier 1985 (1985-01-01) * revendication 6 * --- | 1,7 | |
| A | US 4 648 888 A (R.O.ROWLAND) 10 mars 1987 (1987-03-10) * revendication 1 * --- | 1,7 | |
| A,D | EP 0 860 646 A (LITTON SYSTEMS) 26 août 1998 (1998-08-26) * le document en entier * ----- | 1 | |

**DOMAINES TECHNIQUES RECHERCHES (Int.Cl.7)**

B01D

Le présent rapport a été établi pour toutes les revendications

| Lieu de la recherche | Date d'achèvement de la recherche | Examinateur |
|---|---|---|
| BERLIN | 26 juillet 2001 | Bertram, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 01 40 1272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-07-2001

| Document brevet cité au rapport de recherche | | Date de publication | Membre(s) de la famille de brevet(s) | | Date de publication |
|---|---|---|---|---|---|
| US 4971609 | A | 20-11-1990 | AUCUN | | |
| US 4491459 | A | 01-01-1985 | AUCUN | | |
| US 4648888 | A | 10-03-1987 | US | 4516424 A | 14-05-1985 |
| | | | US | 4561287 A | 31-12-1985 |
| | | | US | 4627860 A | 09-12-1986 |
| EP 860646 | A | 26-08-1998 | US | 5858062 A | 12-01-1999 |
| | | | AU | 723408 B | 24-08-2000 |
| | | | AU | 5296398 A | 13-08-1998 |
| | | | CA | 2228459 A | 10-08-1998 |
| | | | JP | 10225518 A | 25-08-1998 |

EPO FORM P0460

Pour tout renseignement concernant cette annexe : voir Journal Officiel de l'Office européen des brevets, No.12/82